(19) Europäisches Patentamt
European Patent Office
Office européen des brevets

(11) Publication number: **0 064 091**
**A1**

(12) # EUROPEAN PATENT APPLICATION

(21) Application number: 81103415.6

(22) Date of filing: 06.05.81

(51) Int. Cl.³: C 07 C 153/00
C 07 C 131/00, C 07 C 148/00

(43) Date of publication of application:
10.11.82 Bulletin 82/45

(84) Designated Contracting States:
DE FR GB IT

(71) Applicant: ALLIED CORPORATION
Columbia Road and Park Avenue P.O. Box 2245R (Law Dept.)
Morristown New Jersey 07960(US)

(72) Inventor: Chempolil, Thomas Mathew
19 Openaki Road
Randolph New Jersey 07869(US)

(74) Representative: Baillie, Iain Cameron et al,
c/o Ladas & Parry Isartorplatz 5
D-8000 München 2(DE)

(54) **Production of hydrocarbylthioaldoxime in aqueous methanol solvent.**

(57) An aldoxime is reacted with chlorine or bromine to form a haloaldoxime in aqueous solution including methanol, and the haloaldoxime in aqueous solution including methanol is reacted with an alkali hydrocarbylmercaptide, or a hydrocarbylthiol and a base, to form a hydrocarbylthioaldoxime product in aqueous solution including methanol. The product solution is neutralized; methanol is evaporated from the product solution; and the product is recovered as a solid. The product, such as methyl-thioacetaldehyde oxime, is a pesticide intermediate.

EP 0 064 091 A1

Croydon Printing Company Ltd.

## DESCRIPTION

## PRODUCTION OF HYDROCARBYLTHIOALDOXIME IN AQUEOUS METHANOL SOLVENT

### BACKGROUND OF THE INVENTION

The production of hydrocarbylthioaldoximes of the formula (1)

R-C=NOH (1)
  |
 S-R'

where R is alkyl of 1-3 carbons, hydroxyalkyl of 1-3 carbons, phenyl or cyclohexyl and R' is alkyl of 1-3 carbons, phenyl or cyclohexyl is well known. Conventionally, the aldoxime R-CH=NOH is reacted with chlorine or bromine to form a haloaldoxime of formula (2)

R-C=NOH (2)
  |
  X

where X is Br or Cl, with the formation of by-product HCl or HBr. The haloaldoxime is then reacted with either an alkali mercaptide of the formula M-S-R' or with the alkanethiol R'-S-H and the base M-OH, where M is sodium, potassium or lithium to form the product of formula (1). Again, by-product HX is theoretically liberated; however, conventionally, excess base is added, either in forming the alkali mercaptide or with the alkanethiol, so as to neutralize the by-product acid of each step.

While the alkylthioaldoxime of formula (1) may be further reacted in solution (especially if nonaqueous) to form additional products, such as thiocarbamates, it is frequently desirable to isolate the hydrocarbylthioaldoxime. It is desirable to transport same as a dry solid, especially since, in the presence of water and/or acidity, it is capable of hydrolysis, melting and decomposition. One method of recovering the hydrocarbylthioaldoxime from solution is to neutralize the product solution and then cool until most of the hydrocarbylthioaldoxime precipitates, and thereafter separate the precipitate from the mother liquor. The mother liquor presents, however, a disposal problem since it generally contains some dissolved product hydrocarbylthioaldoxime and various by-products including dialkyl disulfides, mercaptan, aldehydes, nitriles, free hydroxylamine and the like, in addition to large amounts of the alkali halide MX. In order to dispose of this liquid in an environmentally safe manner, it has been necessary to heat same to elevated temperatures with acid, phase separate the organics and then steam strip the remaining organics for incineration before seweraging the aqueous salt solution.

Solvents for the above two reactions have been described in the literature. Thus, for example, U.S. Patents 3,658,869, 3,574,736 and 3,987,096 describe such reactions in water. U.S. Patent 3,658,869 requires essentially only water as the liquid reaction medium. U.S. Patent 3,574,736 requires that the aldoxime be present in solution in an amount from 1 to 13 percent by weight. U.S. Patent 3,752,841 requires that the solvent contain at least 10 percent by weight of dimethylformamide. U.S. Patent 3,987,096 requires that the process be continuous or semi-continuous and that the aldoxime, namely acetaldoxime, be at a concentration below 1%. U.S. Patent 3,574,736 indicates the desirability of keeping the temperature range during the chlorination reaction between 25°C and -15°C, preferably near the freezing point of the solution. It also indicates the desirability of adding a water-miscible organic solvent

which does not react with chlorine under reaction conditions to depress the freezing point of the reaction mass, and suggests methanol, dioxane and dimethylformamide.

J.M.J. Tronchet et al. have described in Pharm, Acta Helv, vol. 50, No. 11, pp. 404-414 (1975) various intermediates in the following reaction sequence:

(3) (4) (5)

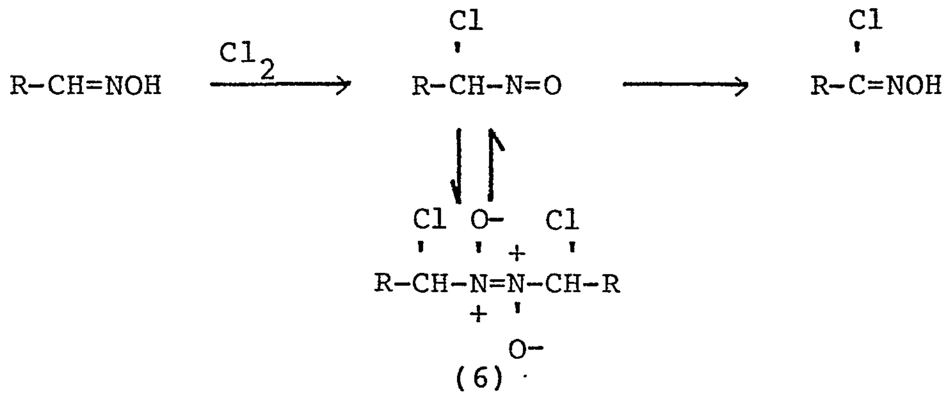


(6)

It will be appreciated that the starting material (3) is an aldoxime and the product (5) is a haloaldoxime included within above formula (2). The compound of primary interest herein is where R is methyl: that is the chlorination of acetaldoxime to chloroacetaldoxime. This article indicates the structure of the "nitroso" intermediate (4) and the "dimer" by-product (6) which is in equilibrium therewith.

It is also known that oximes generally, and including acetaldoxime and chloroacetaldoxime, are capable of side reactions including hydrolysis to aldehydes and hydroxamic acids, respectively, and, in the case of halogenated compounds, further hydrolysis to free acids. It is also known that, in the presence of excess chlorine, chloroacetaldoxime (5) (where R is methyl) or the intermediate nitroso compound (4) can be further chlorinated to 1,1-dichloro-1-nitroso ethane or

$CH_3-CCl_2-N=O$. All of the following products are also capable of explosive decomposition at even slightly elevated temperatures:

(6) The dimer (at about 53°C or above)

(5) Chloroacetaldoxime (acetohydroxamyl chloride) (above about 25°C)

BRIEF DESCRIPTION OF THE DRAWING

Figure 1 is a schematic of one embodiment of the present process.

BRIEF DESCRIPTION OF THE INVENTION

The present invention includes an improvement in a process for preparing a hydrocarbylthioaldoxime of the formula:

$$\begin{array}{c} R\text{-}C\text{=}NOH \\ | \\ S\text{-}R' \end{array}$$

wherein R is alkyl of 1-3 carbons, hydroxyalkyl of 1-3 carbons, phenyl or cyclohexyl and R' is alkyl of 1-3 carbons, phenyl or cyclohexyl by the steps comprising:

(a) reacting an aldoxime of the formula R-CH=NOH in aqueous media with chlorine or bromine to form a first product solution of haloaldoxime of the formula R-CX=NOH where X is Cl or Br in aqueous media, and

(b) reacting the first product solution formed in (a) with a thiohydrocarbylation reagent selected from the group consisting of an alkali hydrocarbylmercaptide and a mixture of an alkali hydroxide with a hydrocarbylthiol to form a second product solution of the hydrocarbylthioaldoxime in aqueous media,

(c) neutralizing the second product solution; and

(d) recovering the hydrocarbylthioaldoxime as a solid from the neutralized second product solution. In the improvement, both reactions are carried out in a solvent consisting essentially of 80-99% water and 1-20%

methanol by weight and methanol is evaporated from the second product solution before the hydrocarbylthioaldoxime is recovered.

The starting material of the present invention is an aldoxime of formula (3) wherein R is alkyl or hydroxyalkyl of 1-3 carbons, phenyl or cyclohexyl. Examples include acetaldehyde oxime, propionaldehyde oxime, n-butyraldehyde oxime, hydroxyacetaldehyde oxime, 4-hydroxybutyraldehyde oxime, benzaldehyde oxime and cyclohexanecarboxaldehyde oxime. Acetaldehyde oxime is preferred. The halogenating agent is chlorine or bromine, preferably chlorine.

The thiohydrocarbylation agent may be an alkali hydrocarbylmercaptide such as the sodium, potassium lithium methylmercaptides, ethylmercaptides, propyl mercaptides, phenylmercaptides and cyclohexyl-mercaptides. Alternatively, an alkali hydroxide such as NaOH, KOH or LiOH may be introduced together with the hydrocarbylthiol such as methanethiol, ethanethiol, propanethiol, benzenethiol or cyclohexanethiol. In either event it is preferred that some excess hydroxide be present: e.g. a small amount on a molar basis with the alkali hydrocarbylmercaptide or a small excess over the molar amount of the hydrocarbylthiol. Thus the thiohydrocarbylation is preferably conducted at a basic pH producing a basic (over pH 7) second product solution.

The reaction temperature for the halogenation can vary from the freezing point of the reaction mixture to the temperature at which the haloaldoxime becomes susceptible to violent decomposition (about 25°C, at least in the case of chloroacetaldehyde oxime). The reaction temperature for the thiohydrocarbylation is subject to similar constraints and to an increasing pressure of mercaptan in some instances if it is volatile (as is methanethiol). In general, the reaction temperatures of the present invention are those of the prior art patents discussed above. The preferred

temperature range for halogenation is between about -15°C and about 10°C. The preferred temperature range for the thiohydrocarbylation is between about 0°C and about 30°C.

The product hydrocarbylthioaldoxime may be recovered from the second product solution by a variety of techniques. It is desirable to first neutralize the second product solution to a moderate pH such as about 6.5 to about 8.0, and preferably to no more than pH 7.5. The methanol may be evaporated from the second product solution before, during or after neutralization. At least with R' being methyl or ethyl, the hydrocarbylmercaptan R'-SH is frequently also evaporated with the methanol. They may be separated by conventional techniques (e.g. distillation) and recycled, e.g. by recycling the methanol to the chlorination step and the mercaptan to either an alkali hydrocarbylmercaptide-forming step or to the thiohydrocarbylation step.

After the methanol has been evaporated and the second solution has been neutralized, the product may then be recovered, preferably by cooling the organic solution to precipitate the product hydrocarbylthioaldoxime. Alternatively, the neutralized aqueous solution may be extracted with an organic solvent such as methylene chloride, toluene or the like to separate the hydrocarbylthioaldoxime from the aqueous system, and then the organic solution concentrated to isolate the solid. The recovered solvent can be recycled to reextract the product from the neutralized aqueous solution. Whether the product is precipitated from the aqueous solution or from an organic solvent, it is preferred to thoroughly dry it for stability purposes, particulary against hydrolysis.

An overall continuous process for the production of solid hydrocarbylthioaldoxime according to the present process is depicted in Figure 1. For simplicity, the simplest product methylthioacetaldehyde oxime (hereinafter MTAAO) is used as the product and the

appropriate raw materials acetaldehyde oxime and methyl mercaptan are shown as reactants introduced into the system.

A first reactor 10 equipped with agitation means is shown. Methanol is introduced into reactor 10 through stream 11 and aqueous acetaldehyde oxime is introduced into reactor 10 through stream 12. The concentration of acetaldehyde oxime and the relative rates in streams 11 and 12 are designed to form a solvent system of 1-20% methanol and 80-99% water, with the acetaldehyde oxime introduced being between about 15 and about 40%, preferably between about 15 and about 30%, of the solvent, by weight. Chlorine is also introduced into reactor 10 through stream 13 at a rate regulated by a variable flow valve 14 controlled as described below.

Reaction mixture is continuously withdrawn from the bottom of reactor 10 through stream 15 and pumped through a meter 16 which determines the presence of free chlorine and then through a heat exchanger to cool the reaction mixture. Meter 16 is operatively connected to variable flow valve 14 so as to admit chlorine at essentially the maximum rate permissible without producing free chlorine in stream 15. After cooling, the fluid in stream 15 is split into stream 17 which is reintroduced into reactor 10 and stream 18 which is carried forward to thiomethylation reactor 20.

Reactor 20 is also equipped with agitation. A conventional system for the production of sodium methyl mercaptide 21 is present so as to produce a stream 22 containing approximately equal molar amounts of free sodium hydroxide and sodium methyl mercaptide $CH_3SNa$. The reaction mass is continuously withdrawn from the bottom of reactor 20 in stream 23 and pumped through a heat exchanger to cool same and then split into a recycle stream 24 which is reintroduced into reactor 20 and a forward stream 25.

Stream 25, now containing the second aqueous product solution of methylthioacetaldehyde oxime (MTAAO) is introduced into neutralization reactor 26, also equipped with agitation, where free acid HCl is mixed therewith coming in through stream 27. Methanol is evaporated from the solution in vessel 26 through stream 28, which also may contain free methyl mercaptan. A separation vessel 29 (e.g. a still equipped with pressure and temperature control and reflux, all being conventional and not illustrated) separates the methyl mercaptan from the methanol by distillation or other physical or chemical process. The methyl mercaptan is returned from vessel 29 in stream 30 to system 21. The methanol is returned from vessel 29 in stream 31 to stream 11 and reintroduced into the chlorination reactor 10.

The effluent from the neutralization vessel 26 is removed in stream 32 and cooled in heat exchanger 33 and then conducted to liquid-solid separation device 34 (a centrifugation system or the like). Solid product MTAAO 35 is removed from separation vessel 34 and then dried. The remaining liquid is removed from separation vessel 34 in stream 36 and conducted to a disposal system 37 which may include acidification of the stream, separation into aqueous and organic phases, evaporation of residual organics in the aqueous phase, incineration of the combined organic phases and the safe disposal of the remaining aqueous sodium chloride solution.

The presence of methanol in the solvent has several desirable effects. In chlorination reactor 10, methanol retains any dimer formed in solution so that it may revert to nitroso compound (based on the equilibrium) and then irreversibly rearrange to the chloroaldehyde oxime. The absence of free dimer as a slurried solid in stream 18 avoids such hazards as explosivity and a source of acidity that causes isomerization and violent MTAAO decomposition during drying.

In addition, the ability of the system to

prevent dimer from precipitating out permits the concentration of acetaldoxime relative to solvent introduced in streams 11 and 12 to exceed the 13% permissible maximum set when water is the sole solvent. Therefore, the concentration of oximes (including acetaldoxime, chloroacetaldoxime and MTAAO) throughout the system can be significantly increased. The result is that the MTAAO is much more concentrated in stream 32 than would be possible if methanol were not introduced into the system. Accordingly, the quantity of liquid in stream 36 sent to disposal system 37 is decreased. This enables higher overall yields of solid product (since stream 36 invariably contains a few percent MTAAO), less heating in system 37 to evaporate the organics and less load on heat exchanger 33, separation vessel 34 and the incineration system portion of disposal system 37. Finally, the evaporation of methanol from vessel 26 into stream 28 causes a cooling of the liquid in vessel 26. This in turn further reduces the cooling load upon heat exchanger 33 in order to cool the slurry in stream 32 sufficiently to maximize solid product 35.

<u>Example 1</u>

Starting Materials:

| | |
|---|---|
| Aqueous acetaldoxime (54% by wt) | 185 g (1.69 moles) |
| Water | 265 g |
| Methanol | 50 mL (40 g) |
| Chlorine | 120 g (1.69 moles) |

A one liter jacketed resin flask was equipped with an over-head agitator and connected to a dry ice-cooled methanol bath. A platinum depolarizer device was employed to indicate level of chlorination. The temperature of the reaction was recorded by a thermometer placed in the reactor.

The acetaldoxime, water and methanol were mixed together and placed in the reactor. This produced a 20% acetaldehyde oxime solution in 10.3-89.7% methanol-water. Agitation and cooling were started. The temperature was brought to -2°C. Chlorine gas was

sparged in from a pre-weighed lecture bottle through a calibrated flow meter.

Chlorine addition was completed over 30 minutes, while the temperature was maintained between -5 and 0°C. The solution was light bluish-colored and there was no indication of any solid dimer being formed.

After holding for 30 minutes with cooling, the chlorination mass was run into sodium thiomethylate solution (excess) prepared from methyl mercaptan and 50% sodium hydroxide. After the addition was completed, a pH of 12.5 was found. Stirring continued for 15 minutes more, and then pH was adjusted to 7.0 with HCl.

A white precipitate of methylthioacetaldehyde oxime separated out. This precipitate was filtered out after cooling to 0°C.

On drying, 118 g of the methylthioacetalde oxime was collected and its melting point was determined to be 90-92°C. This represents about a 77% yield based upon acetaldehyde oxime.

Gas chromatographic analysis showed no new impurities compared to a sample from operation (at about 13%) employing only water as reaction medium.

Example 2

Example 1 was repeated by forming the 20% acetaldehyde oxime solution in methanol-water, adding chlorine and holding for 30 minutes in an identical manner. Likewise, addition of sodium thiomethylate and neutralization were conducted as in Example 1. The slurry was then subjected to vacuum (about 50 mm mercury or 6.7 kPa) for two hours while holding the temperature at about 30°C. The residue was then cooled to 0°C, and the thick slurry that formed was filtered. The solid was then dried to produce 146 g of MTAAO, melting point 90-92°C. The purity level based on gas chromatography was no different from the product of Example 1, but the yield (based on acetaldehyde oxime) was about 95% instead of about 77%.

Example 3

Example 1 was repeated with the following starting materials:

| | |
|---|---|
| Aqueous acetaldehyde oxime (54% by wt.) | 278 g (2.54 moles) |
| Water | 172 g |
| Methanol | 150 mL (119 g) |
| Chlorine | 180 g (2.54 moles) |

The 25% acetaldehyde oxime in 28.4-71.6% methanol-water formed from the first three materials was cooled to -2°C. Chlorine was added over 45 minutes, and the mixture was held at 0°C for 30 minutes. The clear light blue solution was added, with stirring, to sodium thiomethylate solution (350 g, or an excess) keeping the temperature below 20°C. After 15 minutes, HCl was added to lower the pH from 12.9 to 7.5. Methanol was then evaporated off at about 50 mm mercury (6.7 kPa) and about 30°C over two hours. The residual slurry was cooled to -5°C to produce a thick slurry which was filtered. The product collected was dried and weighed as 224 g of MTAAO, melting point 90-92°C. The higher methanol content and oxime concentration had no apparent adverse effect on the product which was produced in about 97% yield, based on acetaldehyde oxime.

What is claimed is:

1. In the process for preparing a hydrocarbylthioaldoxime of the formula:

$$\begin{array}{c} R-C=NOH \\ | \\ S-R' \end{array}$$

wherein R is alkyl of 1-3 carbons, hydroxylalkyl of 1-3 carbons, phenyl or cyclohexyl and R' is alkyl of 1-3 carbons, phenyl or cyclohexyl by the steps comprising:

(a) reacting an aldoxime of the formula R-CH=NOH in aqueous media with chlorine or bromine to form a first product solution of a haloaldoxime of the formula R-CX=NOH where X is Cl or Br in aqueous media;

(b) reacting the first product solution formed in (a) with a thiohydrocarbylation reagent selected from the group consisting of an alkali hydrocarbylmercaptide and a mixture of an alkali hydroxide with a hydrocarbylthiol to form a second product solution of hydrocarbylthioaldoxime in aqueous media;

(c) neutralizing the second product solution; and

(d) recovering the hydrocarbylthioaldoxime as a solid from the neutralized second product solution;

the improvement wherein both reactions are carried out in a solvent consisting essentially of 80-99% water and 1-20% methanol, by weight, and methanol is evaporated from the second solution before the hydrocarbylthioaldoxime is recovered.

2. The process of claim 1 wherein R and R' are methyl and X is Cl.

3. The process of claim 1 or 2 wherein the total concentration of aldoxime and haloaldoxime during step (a) is between about 15 and about 40% by weight of solvent.

4. The process of claim 3 wherein said concentration is between about 15 and about 30% by weight of solvent.

5. The process of claim 1 or 2 wherein the second product solution has a pH of at least about 7 and the second product solution is neutralized to a pH about 6.5 to about 8.0 while the methanol is evaporated.

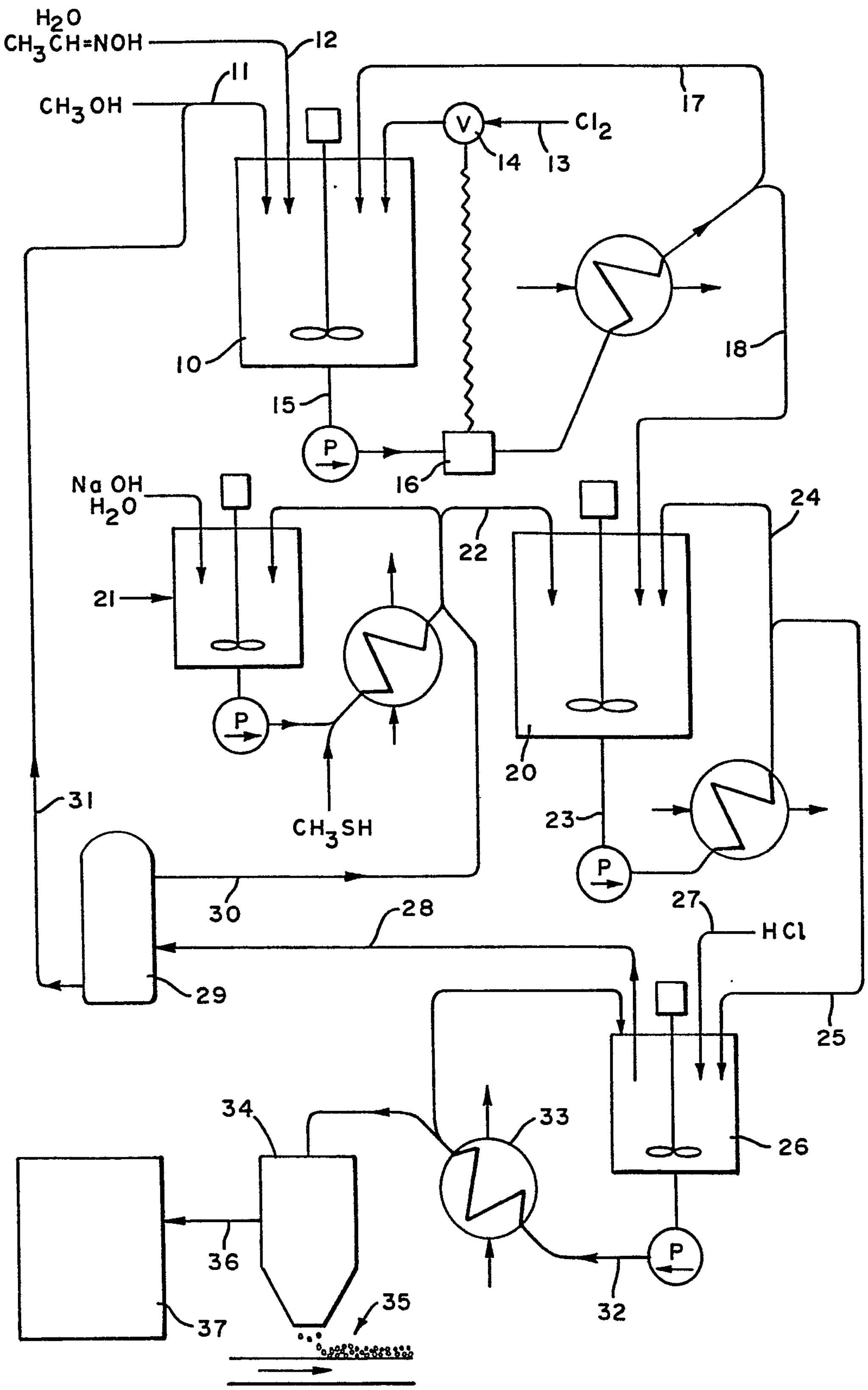
H2O
CH3CH=NOH
12
11
CH3OH
V
Cl2
14
13
17
10
15
18
P
16
Na OH
H2O
22
24
21
P
CH3SH
20
23
P
31
30
28
27
HCl
29
25
34
33
26
P
36
35
32
37

European Patent Office

# EUROPEAN SEARCH REPORT

0064091

Application number

EP 81 10 3415.6

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| | GB - A - 1 262 390 (E.I. DU PONT DE NEMOURS)<br>* claims 1 bis 3 *<br>-- | 1 | C 07 C 153/00<br>C 07 C 131/00<br>C 07 C 148/00 |
| D | US - A - 3 574 736 (J.J. FUCHS)<br>* claim 1; column 2, lines 34 to 41 *<br>---- | 1,2 | |

TECHNICAL FIELDS SEARCHED (Int. Cl.3)

C 07 C 131/00
C 07 C 148/00
C 07 C 149/14
C 07 C 149/26
C 07 C 149/32
C 07 C 153/00

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

Y The present search report has been drawn up for all claims

| Place of search | Date of completion of the search | Examiner |
|---|---|---|
| Berlin | 04-01-1982 | KNAACK |

EPO Form 1503.1 06.78